# EUROPEAN PATENT SPECIFICATION

(11) **EP 0 610 420 B1**
(45) Date of publication and mention of the grant of the patent: **19.04.2000**
(21) Application number: 92924108.1
(22) Date of filing: 16.10.1992
(51) Int. Cl.: C12N 5/06, C07K 14/00

(54) **CELL CYCLE CONTROLLING COMPOSITIONS AND METHODS OF USING SAME**
Zellzyklus- Kontrollzusammensetzungen und Methoden diese zu benutzen.
COMPOSITIONS ET PROCEDES DE REGULATION DU CYCLE CELLULAIRE

(30) Priority: 17.10.1991 US 778510
(43) Date of publication of application: 17.08.1994
(73) Proprietor: THE REGENTS OF THE UNIVERSITY OF CALIFORNIA, Oakland, California 94612-3550 (US)
(72) Inventor: LEE, Wen-Hwa, San Antonio, TX 78233 (US); LEE, Eva, Y., H., P., San Antonio, TX 78233 (US); GOODRICH, David, W., San Antonio, TX 78245 (US); WANG, Nang-Ping, Seattle, WA 98115 (US)
(74) Representative: Voelker, Ingeborg Carla Emmy
(86) International application number: US9208918
(87) International publication number: WO9308267

(56) References cited:
- EP-A- 0 259 031
- WO-A-89/06703
- WO-A-91/09114
- PROCEEDINGS OF THE NATIONAL ACADEMY OF SCIENCES OF USA. vol. 88, no. 7, 1 April 1991, WASHINGTON US pages 2648 - 2652 SARAH E. FUNK ET AL. 'The Ca2+-binding glycoprotein SPARC modulates cell cycle progression in bovine aortic endothelial cells'
- NATURE vol. 329, 15 October 1987, LONDON GB pages 642 - 645 WEN-HWA LEE ET AL. 'The retinoblastoma susceptibility gene encodes a nuclear phosphoprotein associated with DNA binding activity'
- CELL vol. 67, 18 October 1991, CAMBRIDGE, NA US pages 293 - 302 DAVID W. GOODRICH ET AL. 'The retinoblastoma gene product regulates progression through the G+ phase of the cell cycle'

## Description

### Technical Field

This invention relates in general to the control of cell cycle progression and it more particularly relates to compositions and methods for regulating cell cycle progression to suppress tumorigenesis.

### Background Art

It is well recognized that in the development of higher organisms, such as animals and humans, orderly cell cycle progression is a critical factor. Because of such progression, ordered and systematic cell differentiation occurs so that, ultimately from a single undifferentiated cell, a highly structured organism, having a variety of specialized tissues, develops. In general, physiologically normal cell cycle progression is very important to the organism, not only during the stages of early growth, but throughout the entire life of the organism. Thus, even after the organism has reached maturity, normal cycle progression is still a very important aspect of health. This, for example, can be clearly seen in the importance of proper cellular regulation in the blood forming and reproductive organs.

Under certain circumstances, normal cell cycle progression fails, often with catastrophic effects for the organism. Such a failure is seen, for example, in the various forms of cancer where, because of an unchecked, and uncontrolled, progression of cells through the cell cycle, from the completion of mitosis through interphase and back into mitosis, tumorigenesis becomes, in many cases, a life threatening event. Thus, a restraint on uncontrolled cell cycle progression is sometimes attempted in an effort to treat or control the tumorigenic condition.

Under current conditions, where tumorigenesis has developed, drastic treatment measures, such as radiation therapy and chemotherapy are employed. Both of these modalities are very expensive and, in some cases, very damaging to the organism. Chemotherapy, for example, can cause the death of the patient and when chemotherapy is successful in controlling tumorigenesis, it may have long ten adverse effects on the body. Such an adverse effect may result in premature heart problems for the patient. Thus, while conventional therapeutic methods, such as radiation and chemotherapy, serve a useful purpose in destroying certain uncontrolled cells, they also produce the unwanted effects of destroying useful cells and weakening organ systems.

In view of the foregoing, it would be highly desirable to have techniques and compositions for regulating unwanted cell progression, such as in tumorigenesis, while maintaining cell viability. In addition, it would be further very highly desirable if such techniques and compositions produce little or no adverse effects on the cell and, halt the development of tumorigenesis without causing irreversible changes at the cellular level.

A significant advantage of such novel techniques and compositions, in the case of tumorigenesis, would be the possibility of regulating unwanted cell proliferation, for enough time for other cancer treating procedures to be applied. Thus, the use of such techniques could reduce or eliminate the ravaging effects of conventional radiation and chemotherapeutic treatments, while arresting tumorigenesis. Hence, the patient would retain strength and vigor so that conventional techniques, such as surgery, could be more safely utilized.

The retinoblastoma (RB) gene was known as a tumor suppressor gene, and the absence of RB protein was known to be oncogenic (NATURE 1987, 329 : 642). The function of the RB protein to restrict cell proliferation was known from WO 91/09114. The therapeutic administration of the RB protein to individuals carrying a defective allele of the RB gene had been suggested in EP-A-0 259 031. In WO 89/06703, the treatment of such patients by administering a pharmaceutical composition containing a fragment of the RB protein had been suggested.

### Disclosure of Invention

It is an object of the present invention to provide a cell cycle controlling composition for controlling the progression of the cell cycle in living organisms.

It is a further object of the present invention to provide a safe and effective composition for reversibly arresting cell cycle progression in organisms.

It is a still further object of the present invention to provide a technique which may be used in combination with therapeutic methods to arrest tumorigenesis in organisms.

The present invention relates to the use of a C-terminal biologically active fragment of p110^{RB} of 56 kD for the preparation of a medicament for controlling cell cycle progression in a cell lacking a functional retinoblastoma tumor suppressor gene.

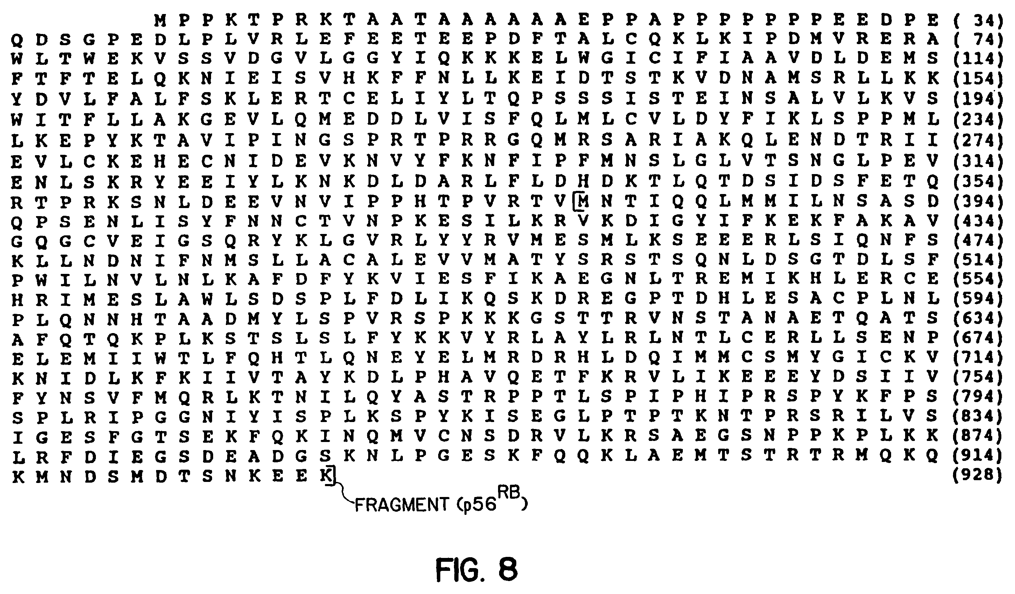

In a preferred embodiment, said C-terminal fragment comprises amino acid 379 to amino acid 928 as shown in Figure 8.

In a more preferred embodiment, the medicament is for the treatment of a pathology characterized by cell cycle progression through the absence of a functional retinoblastoma tumor suppressor protein.

The medicament can be used for reversibly arresting cell cycle progression in organisms or may be used in combination with therapeutic methods to arrest tumorigenesis in organisms.

Furthermore, the medicament can reversibly alter the cell cycle progression of the cell while maintaining its viability. The protein fragments have been found to have the unexpected and surprising characteristic of being soluble in low concentration of glycerol, thereby enhancing their value in pharmaceutical applications.

The invention also relates to a method of controlling cell cycle progression in a cell in vitro, the cell lacking a functional retinoblastoma tumor suppressor gene, comprising in vitro introducing into the cell to be controlled a C-terminal biologically active fragment of p110^{RB} of 56 kD during the G phase of the cell cycle.

In a preferred embodiment, said C-terminal fragment comprises amino acid 379 to amino acid 928 as shown in Figure 8.

An advantage of the present invention is that cell cycle progression can be reversibly arrested in a convenient and safe manner, without insult to the organism. Thus, tumorigenesis may, for example, be controlled.

A further advantage of the present invention is that the composition or medicament utilized for cell cycle progression control is relatively inexpensive and readily obtainable.

A still further advantage of the present invention is the fact that the medicament therein utilized possesses little or no toxic effects on healthy cells, and may be used in conjunction with other methods of cancer treatment.

An even still further advantage of the present invention is the fact that the medicaments are compatible for use with the regulatory regimens and are physiologically compatible with other methods and devices for regulating certain physiological processes of the body such as blood cell production and gamete production.

### Brief Description of Drawings

The above mentioned and other objects and features of this invention and the manner of attaining them will become apparent, and the invention itself will be best understood by reference to the following description of the embodiment of the invention in conjunction with the accompanying drawings, wherein:
FIG. 1 depicts SDS-PAGE analysis of the concentration and purity of protein preparations used for microinjection;
FIGS. 2A-2F are photomicrographs depicting microinjection and immunostaining of labeled Saos-2 cells;
FIG. 3 graphically depicts the percentage of labeled cells up to four days after injection;
FIG. 4 graphically depicts the percentage of labeled cells as a function of p56^{RB} concentration;
FIG. 5 graphically depicts the effect on cells when p56^{RB} is injected in S phase;
FIG. 6A graphically depicts the dependence of cell cycle arrest on the time period of injection of p56^{RB};
FIG. 6B graphically depicts the time of onset of S phase in Saos-2 cells after release from nocodazole arrest in mitosis;
FIG. 6C graphically depicts SAOS-2 cells coinjected with p56^{RB} and R or G after release from nocodazole treatment;
FIG. 7 is a composite of FIGS. 7A, 7B, 7C, and 7D which depict the nucleotide sequence of the retinoblastoma gene and the amino acid sequence of the RB protein;
FIG. 8 depicts the amino acid sequence of the RB protein;
FIG. 9 depicts the nucleotide sequence of the retinoblastoma gene and the amino acid sequence of the RB gene product and, in addition, depicts the p56^{RB} portion of the RB protein; and
FIG. 10 graphically depicts various stages of the cell cycle and the status of RB protein point in the cell cycle.

### Best Mode for Carrying Out the Invention

It is generally recognized that, after mitosis of a somatic cell is completed, at the end of telophase, the cell enters into an interphase which, depending on a variety of factors, may be of short duration or last for a long period of time. Thus, for example, after cell differentiation and development of nerve tissue, the nerve cells may have a very long interphase. Conventionally, cellular interphase may be regarded as having three stages: G1 in which cell growth occurs without DNA replication, S phase, in which DNA replication occurs, and G2, in which DNA replication has been completed and the cell prepares for division. As hereinafter described in greater detail, certain gene protein products, or fragments thereof, have the capacity for controlling progression through the cell cycle by stopping reversibly the progression at G1. According to the present invention, a C-terminal biologically active fragment of p110^{RB} of 56 KD, in particular the C-terminal fragment comprising amino acids 379 to 928 as shown in Figure 8, was used for the preparation of a medicament for controlling cell cycle progression in a cell lacking a functional retinoblastoma tumor suppressor gene and for controlling cell cycle progression in a cell in vitro, wherein the cell cycle controlling compositions were introduced into cells during the interphase portion of their cell cycles, to cause a reversible alteration of the cell cycle progression, while maintaining cell viability. After a certain time, when the compositions degraded sufficiently within the cell, the cell cycle has been observed to be reinstated with the cell progressing toward subsequent stages of interphase.

With regard to pathological conditions in an organism, such as tumorigenesis, there is a manifestation of unwanted cell cycle acceleration, in some cells. As more fully discussed below, cancer suppressor gene protein products, such as the RB protein, or a fragment thereof, were utilized to arrest Saos-2 osteosarcoma cells in the G1 stage of interphase. It has been found that the administration of the protein to the cells had no toxic effect on the cells, and was reversible.

In the examples discussed below, the following experimental procedures were followed.

### Cell Culture

Saos-2 cells, an osteosarcoma cell line, were obtained from American Type Culture Collection. SR-40, which stably expresses exogenous, wild-type RB protein, was derived from Saos-2 by single cell cloning after infection with a retrovirus carrying the RB gene. Huang, H.-J. S., Yee, J.-K., Shew, J.-Y., Chen, P.-L., Bookstein, R., Friedmann, T., Lee, E.Y.-H.P. and Lee, W.-H. (1988) Science 242, 1563-1566. African Green monkey kidney cell lines CV-1 and COS-7 were also obtained from American Type Culture Collection. COS-7 was derived from CV-1 by transformation with an origin-defective SV-40. Gluzman, Y. (1981) Cell 23, 175-182. All the cells were cultured in Dulbecco's modified Eagle's medium plus 10% fetal calf serum as recommended.

### Synchronization of Cells

Cells were synchronized at the G1/S boundary by isoleucine starvation for 36 hours and then incubated in complete medium supplemented with either aphidicolin or hydroxyurea (both from Sigma) for an additional 12 or 16 hours respectively. Heintz, H.H. and Hamlin, J.L. (1982) Proc. Natl. Acad. Sci. USA. 79, 4083-4087. Cells were released from G1/S block by washing three times with phosphate buffered saline (PBS), and re-feeding with complete media. Metaphase arrested cells were collected after an 8 to 12 hours treatment with 0.04 µg/ml nocodazole (sigma) as previously described. Zieve, G.W., Turnbull, D., Mullins, J.M. and McIntosh, J.R. (1980) Nocodazole Exp. Cell Res. 126, 397-405. To increase the yield of mitotic cells, cells were first arrested at the G1/S boundary by a 12-hour treatment with aphidicolin. Six hours after the removal of aphidicolin, nocodazole was added to the medium. Mitotic cells were collected by gentle washing and shaking and were then replated onto 35 mm dishes. Six hours after seeding, the unattached cells were washed away and cells were injected at various times thereafter.

### Protein Preparation

p56^{RB} is the C-terminal half of the RB protein and contains both regions essential for SV40 T-antigen binding. It is produced in *E.coli* from a T7 RNA polymerase expression system. Huang, S., Lee, W.-H. and Lee, E.Y.-H.P. (1991) Nature 350, 160-162. p110^{RB} is produced in insect cells by the recombinant baculovirus technique. Wang, N.P., Qian, Y., Chung, A.E., Lee, W.-H. and Lee, E.Y.-H.P. (1990b) Cell Growth & Differ. L, 429-437. Both proteins were purified to homogeneity by conventional chromatography. Histone H1 and Rabbit anti-goat IgG were purchased from Boehringer Mannheim and Vector laboratories, respectively. Antibodies .495, .47 and R2 were concentrated in microinjection buffer to an approximate concentration of 1 mg/ml. The T-antigen and p53 peptides were the gift of Nicholas Lin. These antibodies are described in Huang, S., Wang, N.-P., Tseng, B.Y., Lee, W.-H. and Lee, E.Y.-H.P. (1990) EMBO J.9, 1815-1822. The T peptide comprises amino acids 101-118, and was dissolved in microinjection buffer at 1mM or 5mM. Ludlow, J.W., deCaprio, J.A., Huang, C.-M., Lee, W.-H., Paucha, E. and Livingston, D.M. (1989) Cell. 56, 57-65. The mutant T peptide contains a lysine to aspartic acid substitution and was used at 5 mM. Ludlow, J.W., deCaprio, J.A., Huang, C.-M., Lee, W.-H., Paucha, E. and Livingston, D.M. (1989) Cell 56, 57-65. The p53 peptide was is dissolved in microinjection buffer at 5 mM. Protein preparations, except the full-length RB protein, were concentrated in an injection buffer containing 20 mM Tris, pH 7.4; 0.1 mM EDTA; 10 mM KC1; 1 mM 2-mercaptoethanol and 2% glycerol using the Centricon 30 micro-concentrator (Amicon). p110^{RB} was kept in a buffer containing 10% glycerol to reduce aggregation.

Protein preparations were analyzed by SDS-PAGE using standard techniques. Injections were performed directly on cells growing on 35mm culture dishes using an Eppendorf micromanipulator and microinjector with femtotip capillary micropipets. Injection pressure was typically set between 50-100 hPa with an injection time of .3-.5 seconds. It was estimated that the protein preparations were diluted approximately 20 to 50-fold upon injection. Assuming a typical cell volume of 50-100 picoliters and a p56^{RB} concentration of .5-1 milligram per milliliter, about 5-50 million molecules were injected per cell.

After injection, the growth media was supplemented with BrdU (Amersham) according to manufacturer's recommendations. Following the appropriate labeling period, media was removed and cells were washed 3x with PBS. Cells were then fixed by incubation with ice-cold, absolute methanol for 30 minutes. After re-hydration by washing with PBS, specimens were incubated with a mouse monoclonal antibody directed against BrdU (Amersham) for one hour at room temperature. After washing 3x with PBS, a fluorescein conjugated anti-mouse antibody (Amersham) was added and incubation was continued for an additional hour at room temperature. The anti-mouse antibody was removed by washing 3x with PBS, and the specimens were incubated with Texas Red conjugated streptavidin (Amersham). The streptavidin bound to the biotinylated RαG co-injected with all protein preparations and thus served as a cytoplasmic marker for injected cells. After a final wash, a solution composed of equal volumes of glycerol and PBS was added and the specimens were covered with a glass coverslip. Specimens were examined under a fluorescent microscope with Texas Red and Fluorescein filters.

The following examples demonstrate the experimental results achieved after introduction of gene product proteins into tumor cell lines. The capacity of the proteins, or fragments thereof to arrest cell cycle progression in G1 has been demonstrated. In addition, the examples demonstrate that the effect of the blocking occurs only when the protein is administered during the G phase and that the blocked cell cycle progression can be relieved by SV40 T antigen.

In certain specific examples, the RB gene product was used to arrest the cell cycle progression wherein an Saos-2 osteosarcoma cell line was treated with the RB protein or a fragment thereof. It was discovered that cell cycle progression was arrested in G1 and that this progression is reversible.

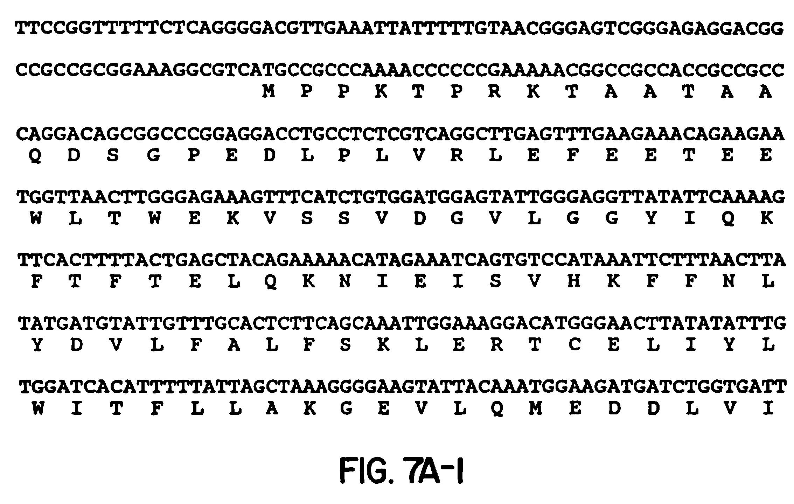

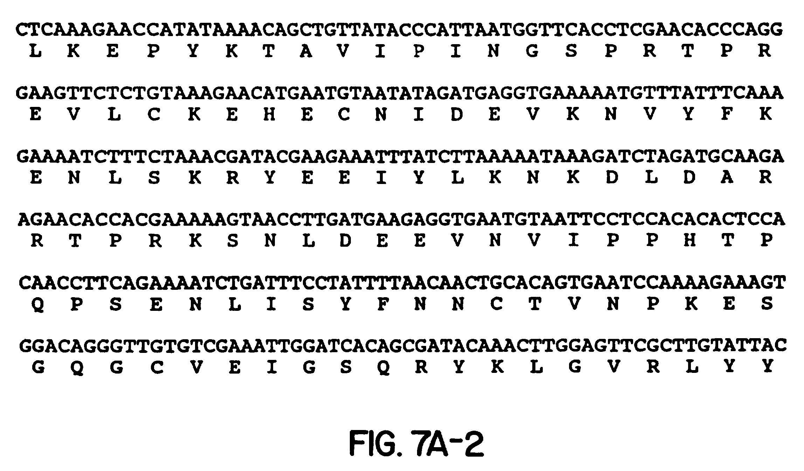

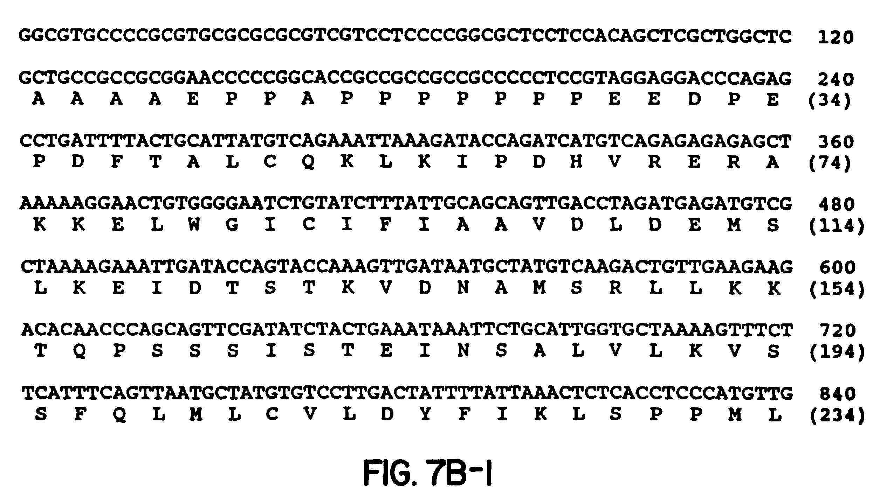

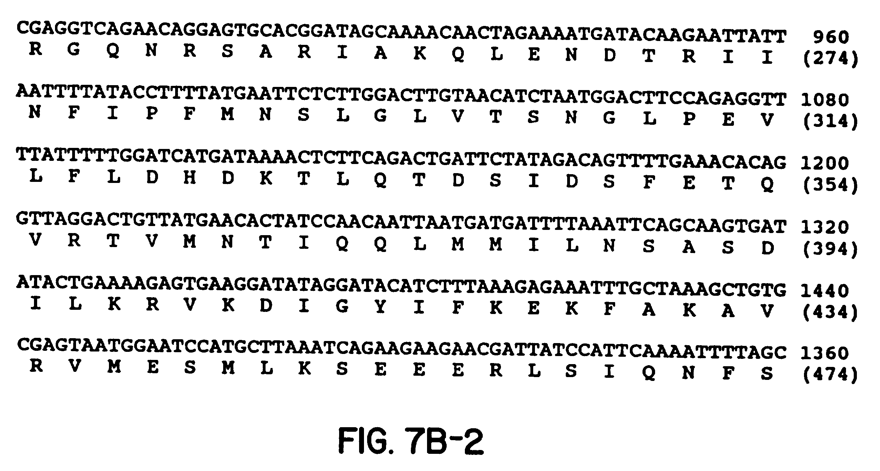

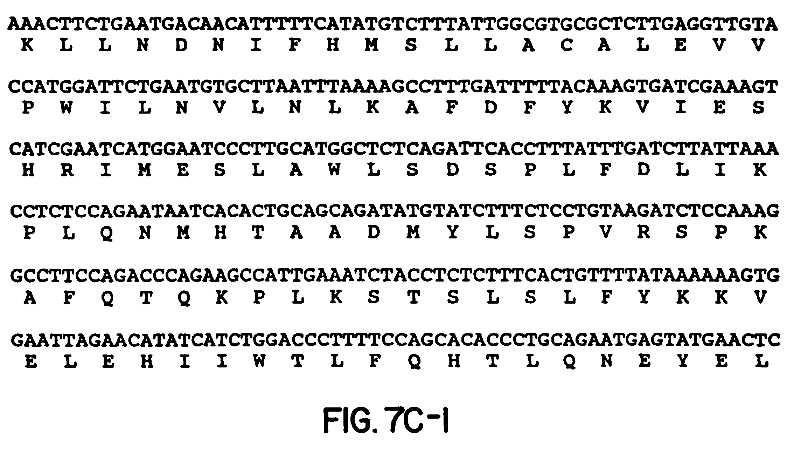

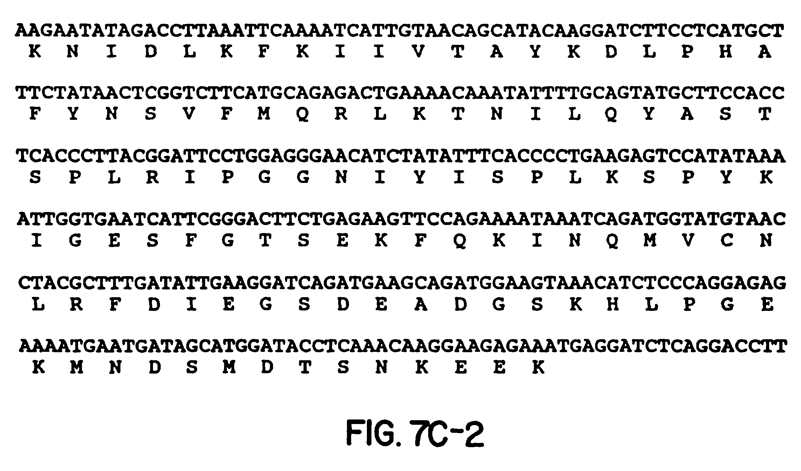

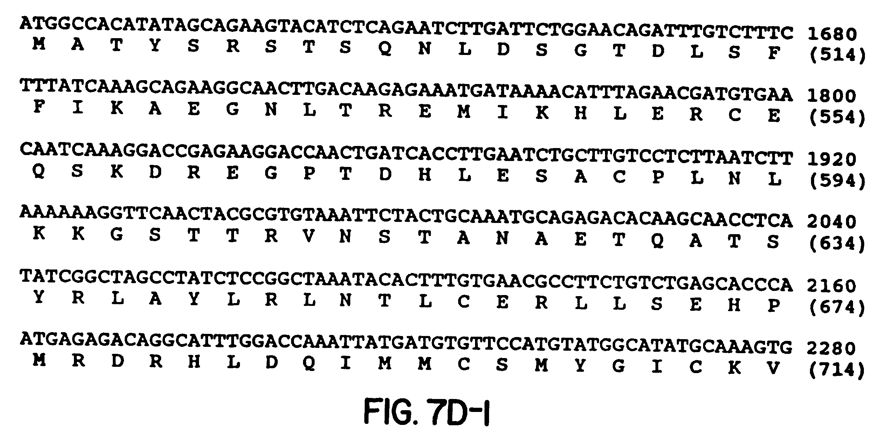

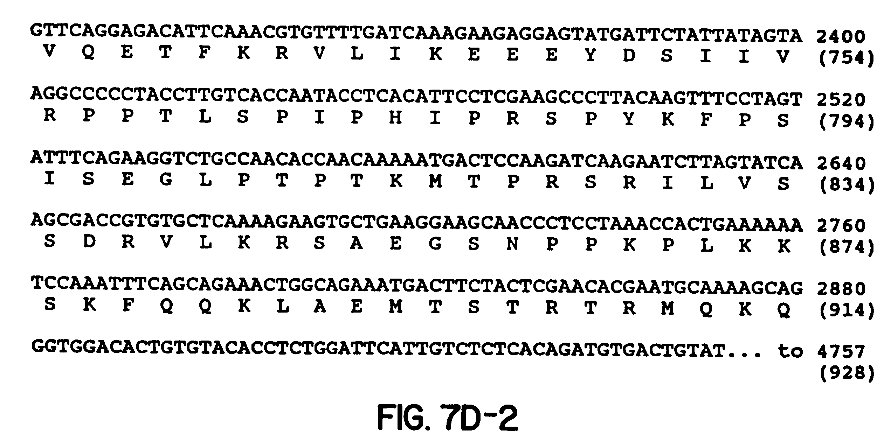

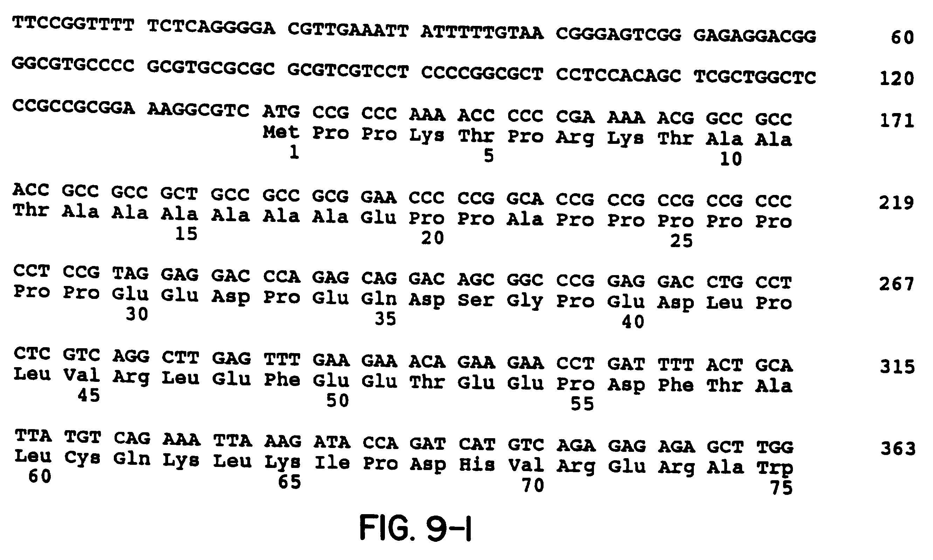

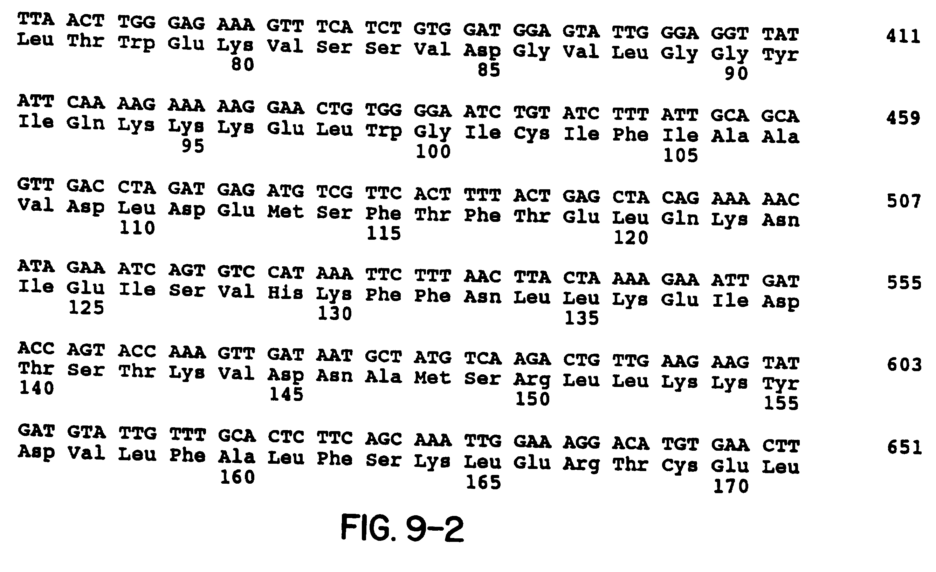

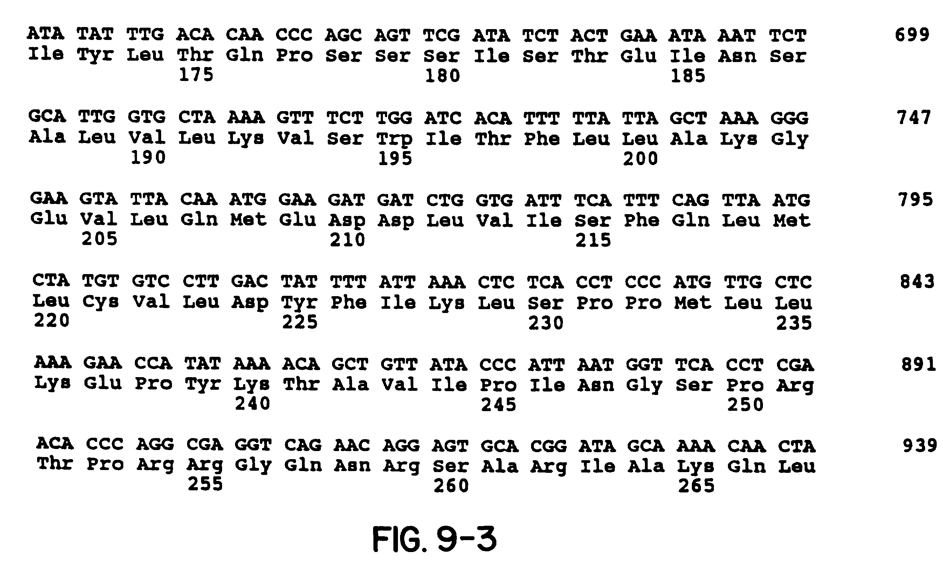

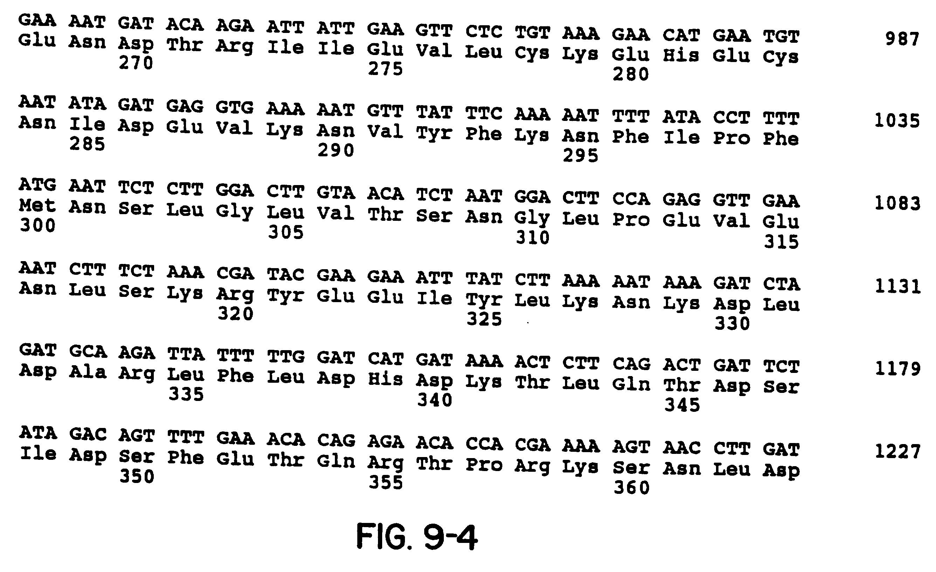

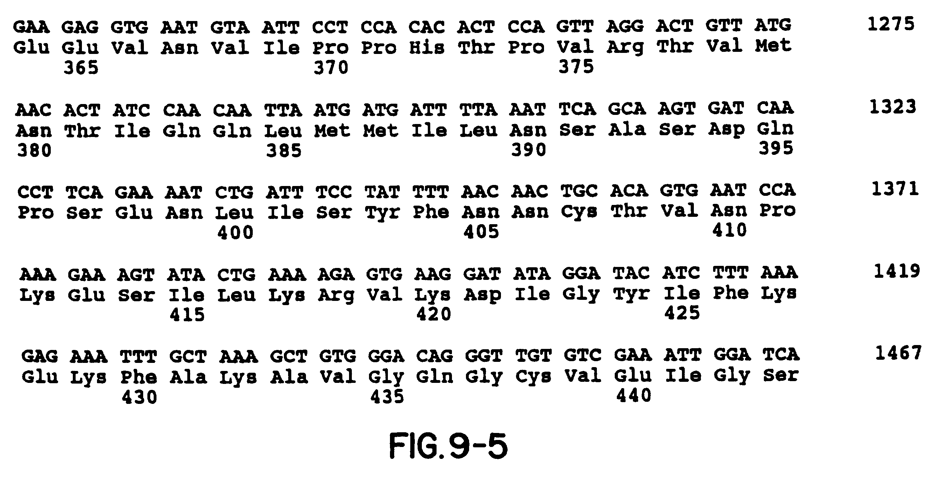

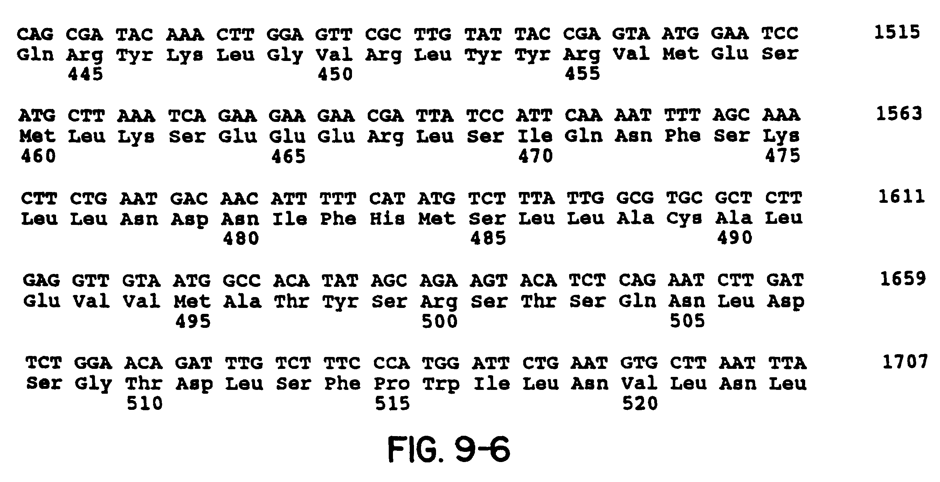

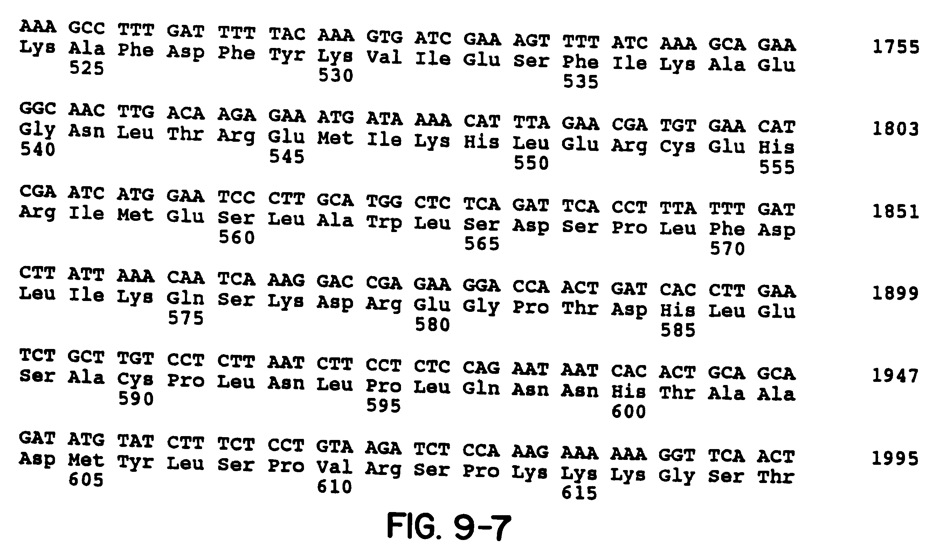

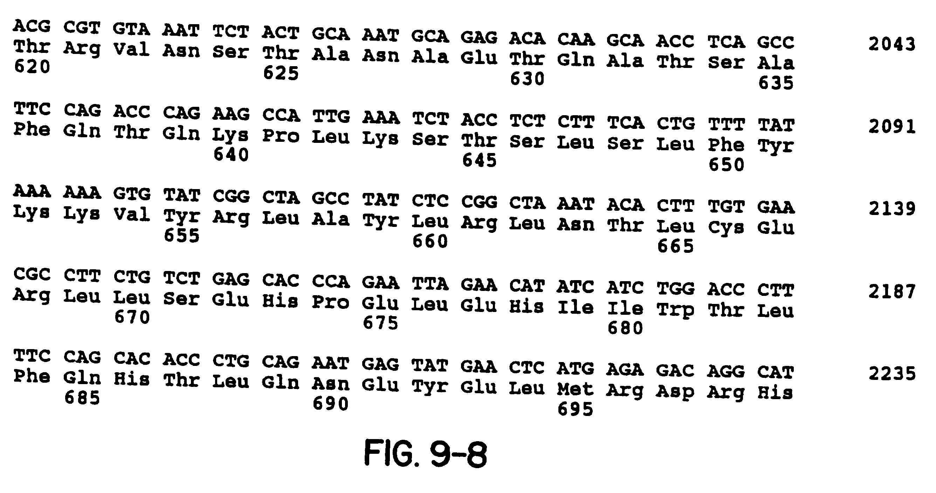

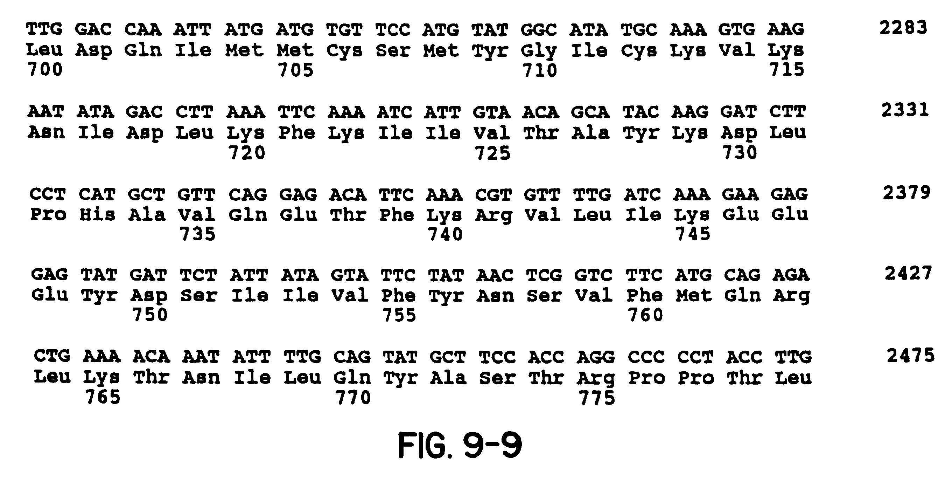

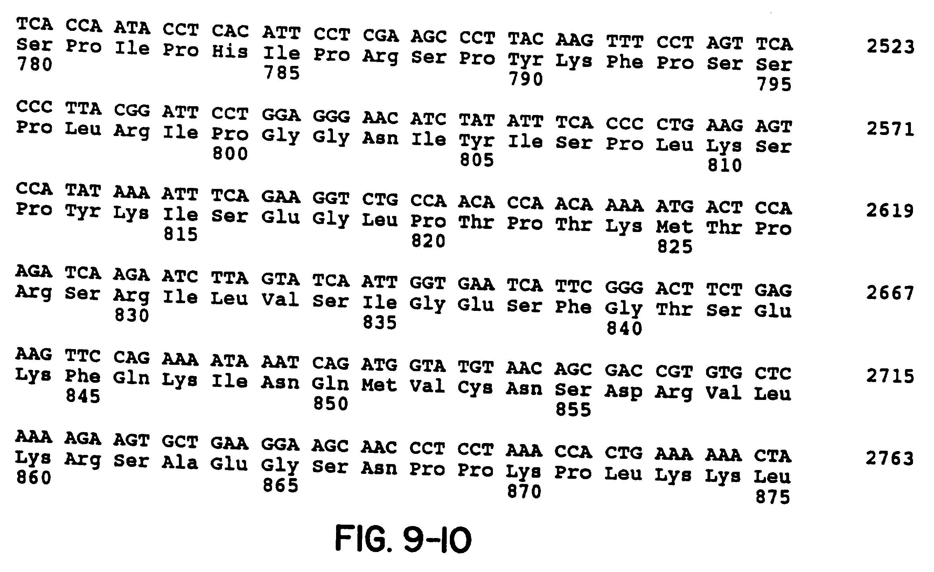

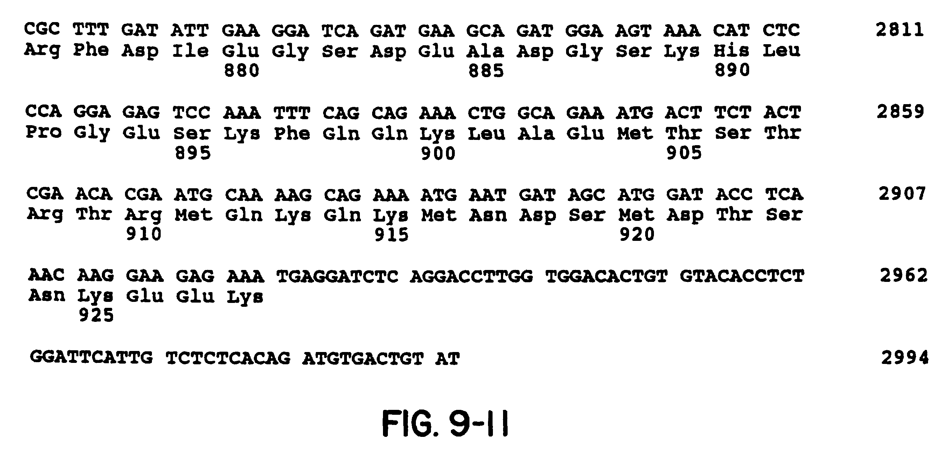

In the following examples, when the retinoblastoma gene is referred to, it is intended to mean the gene having the nucleotide sequence depicted in FIGS. 7 and 9. When reference is made to the RB protein (pRB¹¹⁰) the protein having the amino acid sequence depicted in FIGS. 7, 8 and 9 is intended. With reference to FIG. 8, the truncated protein fragment, p56^{RB} is illustrated. In addition, the p56^{RB}, fragment is depicted in FIG. 9 as commencing at arrow A and terminating at arrow B. With further reference to FIG. 9, the C terminal peptide is illustrated, commencing at amino acid 917 (arrow C) and terminating at amino acid 928 (arrow B).

Single-letter abbreviations in the drawings for the amino acid residues are: A, Alanine; C, Cysteine; D, Aspartic acid; E, Glutamic acid; F, Phenylalanine; G, Glycine; H, Histidine; I, Isoleucine; K, Lysine; L, Leucine; M, Methionine; N, Asparagine; P, Proline; Q, Glutanine; R, Arginine; S, Serine; T, Threonine; V, Valine; W, Tryptophane; and Y, Tyrosine.

### EXAMPLE I

### Introduction of RB Proteins into Human Osteosarcoma Saos-2 Cells by Microinjection

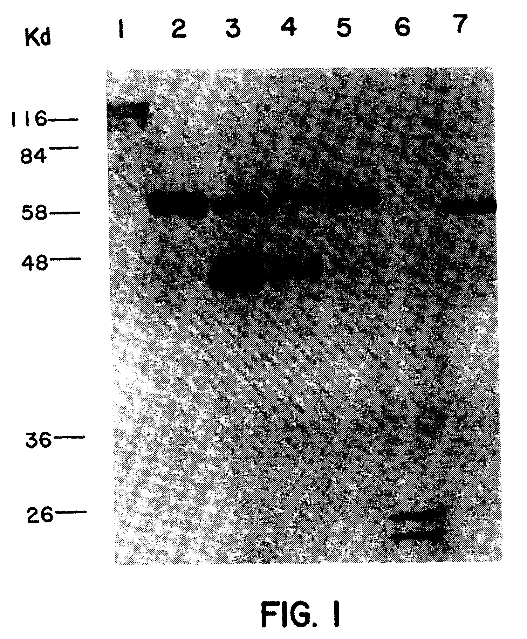

With reference now to FIG. 1, there is shown the concentration and purity of protein preparations used in the microinjection techniques discussed below. The protein preparations used for microinjection were analyzed by SDS-PAGE. Lane 1: p110^{RB} from insect cells infected with recombinant RB baculovirus; lane 2: biotinylated rabbit anti-goat antibody; lane 3: anti-RB .495 antibody; lane 4: anti-RE R2 antibody; lane 5: anti-RB .47 antibody; lane 6: histone H1; lane 7: p56^{RB} from E. coli. One microliter of each sample was loaded on a 15% acrylamide gel. The gel was stained with Coomassie brilliant blue. The positions of molecular weight standards, in kiloDaltons, are indicated.

Two forms of RB protein were prepared for the microinjection experiments. Wild type p110^{RB}, both hypophosphorylated and unphosphorylated forms, was purified to near homogeneity from baculovirus infected insect cells. At concentrations approaching 1 mg/ml, however, the protein aggregated into a form which could not be injected. To partially alleviate this problem, p110^{RB} was purified, stored, and injected in buffers containing 10% glycerol. An unphosphorylated, amino-truncated 56 kDa RB protein (p56^{RB}), containing an intact T-antigen binding domain, was expressed in E. coli and purified to near homogeneity. Since p56^{RB} could be concentrated to 1 mg/ml and injected in a standard buffer containing 2% glycerol, it was used in most of the following experiments.

Human osteosarcoma cell line Saos-2 was chosen as the recipient cell line. This cell line lacked expression of wild-type p110^{RB}, but contained a cytoplasmic, carboxy-truncated 95 kDa protein which can not bind T-antigen. Saos-2 cells responded to exogenous expression of RB, introduced by retrovirus mediated gene transfer, by an initial enlargement of cell size and loss of tumorigenicity in nude mice. Hence, it was determined that these cells might be particularly sensitive to injection of RB protein.

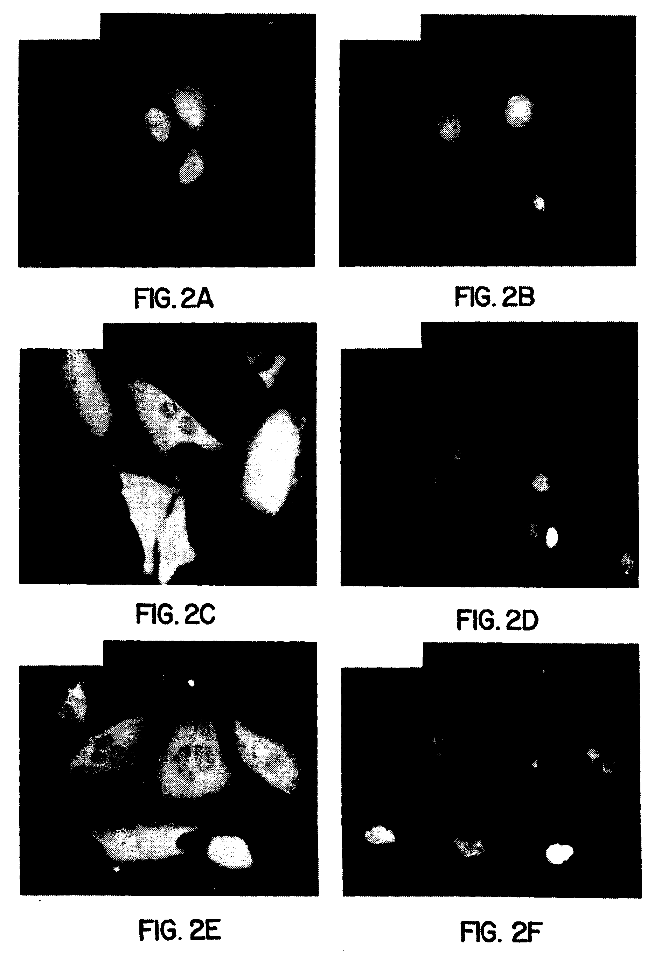

In FIG. 2, there is depicted the results of microinjection and immunostaining of Saos-2 cells labeled with BrdU. Saos-2 cells were injected as described herein. Panel A. shows cells injected with p56^{RB}, immediately fixed, and indirectly immunostained for RB protein with Texas Red. Panel B contains uninjected cells labeled with BrdU for 4 hours, and then fixed and immunostained with fluorescein conjugated anti-BrdU antibody. Panels C and D are a single field of synchronized cells co-injected in early G1 with p56^{RB} and RαG, incubated with BrdU for 24 hours, then fixed and stained. The cells stained with Texas Red mark injected cells, while the cells stained with fluorescein indicate cells which have incorporated BrdU. Panels E and F are a single field of cells also injected in early G1 with RαG alone.

To determine if the proteins could be translocated to the nucleus, sub-confluent, asynchronously growing Saos-2 cells were cytoplasmically microinjected with p56^{RB} or p110^{RB} and fixed 5 to 15 minutes later. The cells were then immunostained with rabbit anti-RB antibody .47 and a Texas Red conjugated anti-rabbit antibody. Staining was mainly observed in the nucleus (FIG. 2A), although there was some staining observed in the cytoplasm of some injected cells. Both p110^{RB} and p56^{RB} proteins were capable of being transported to the nucleus within 15 minutes.

For subsequent experiments, cells were typically co-injected with an RB protein and a biotinylated, polyclonal rabbit anti-goat antibody (RαG) that served as a cytoplasmic marker for injected cells. It was estimated that 5-50 million molecules of RB protein were injected per cell. The number of endogenous RB protein molecules per cell was estimated to be approximately 1 million. Hence the injected protein represented at least a 5 to 50-fold excess over endogenous levels. Following injection the cells were incubated in growth media containing bromodeoxyuridine (BrdU). Cells progressing through S phase during the labeling period will incorporate BrdU into their DNA. After fixation, cells were immunostained for BrdU with a fluorescein-conjugated antibody and for RαG with Texas Red conjugated streptavidin (FIG. 2). The percentage of injected cells that incorporated BrdU could be determined under a fluorescence microscope as the fraction of Texas red-positive cells that were also fluorescein-positive.

BrdU incorporation in RB-injected, asynchronously growing cells was only slightly less than that of uninjected cells or cells injected with RαG alone over a four hour labeling period (Table 1). These results indicated that the RB protein preparations used were not generally toxic to cells as determined by their continued viability, DNA incorporation, and cell adherence.

### EXAMPLE II

### RB Proteins Injected in Early G1 Block Incorporation of BrdU

Since only a small effect on BrdU incorporation was observed upon injection of RB protein, the cells might only be sensitive to RB protein at a particular point in the cell cycle. The effect of RB protein could be more readily observed by injecting synchronized cells at this point. Cells were synchronized by treatment with nocodazole, which arrests cells in mitosis, and were then released and incubated for an additional 6 hours at which time non-adherent cells were removed and the remaining cells injected. Following a 24 hour incubation in the presence of BrdU, cells were fixed and stained for BrdU and RαG. At least 80-90% of uninjected cells could be stained for BrdU. Cells injected with p56^{RB}, however, were almost completely inhibited from progressing through G1 and into S phase over the labeling period. In this regard, please see Table 2 and FIGS. 2C and 2D. Similar inhibition was observed upon injection with p110^{RB}. In contrast, 60-70% of cells injected with histone and RαG, or RαG alone were able to enter S phase during the labeling period (FIGS. 2E and 2F). Although p110^{RB} inhibited BrdU incorporation under these conditions, results were somewhat variable. Over time, p110^{RB} preparations tended to aggregate which led to difficulty in injection. Relative to preparations of p110^{RB}, p56^{RB} activity was very consistent.

To extend this observation to cells which already expressed wild-type p110^{RB}, p56^{RB} was injected into cell line SR-40 which was derived from Saos-2 and stably expressed p110^{RB}. The effect of RB protein injection at early G1 phase on synchronized SR-40 cells was identical to the effect on Saos-2 cells (Table 2); very few cells injected with p56^{RB} entered S phase over the 24 hour labeling period. Thus, the presence of endogenous wild-type p110^{RB} did not interfere with the effect of p56^{RB} on cell cycle progression.

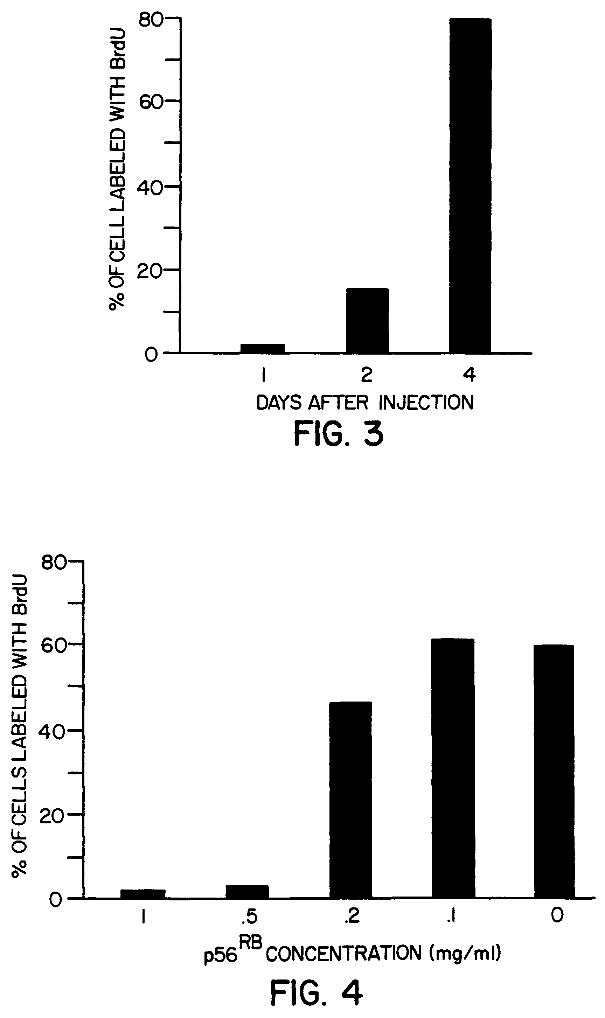

To determine how long the p56^{RB} block to entry of S phase lasted, the labeling period in the presence of BrdU was extended from one day to 2 or 4 days. FIG. 3 graphically shows the number of injected Saos-2 cells which incorporated BrdU during the labeling period. Saos-2 cells were co-injected with p56^{RB} and RαG 6-7 hours after release from nocodazole treatment. After injection, cells were incubated in media supplemented with BrdU for the indicated number of days before fixing and staining. The percentage value indicates the number of injected cells which had incorporated BrdU during the labeling period. The values represent at least 100 injected cells. Inhibition of cell cycle progression was still observed after 2 days of incubation with BrdU prior to fixing and staining. Four days after injection, however, 80% of p56^{RB} injected cells were able to incorporate BrdU. These observations indicated that the block of cell cycle progression by RB protein was reversible, and lasted between 2 and 4 days.

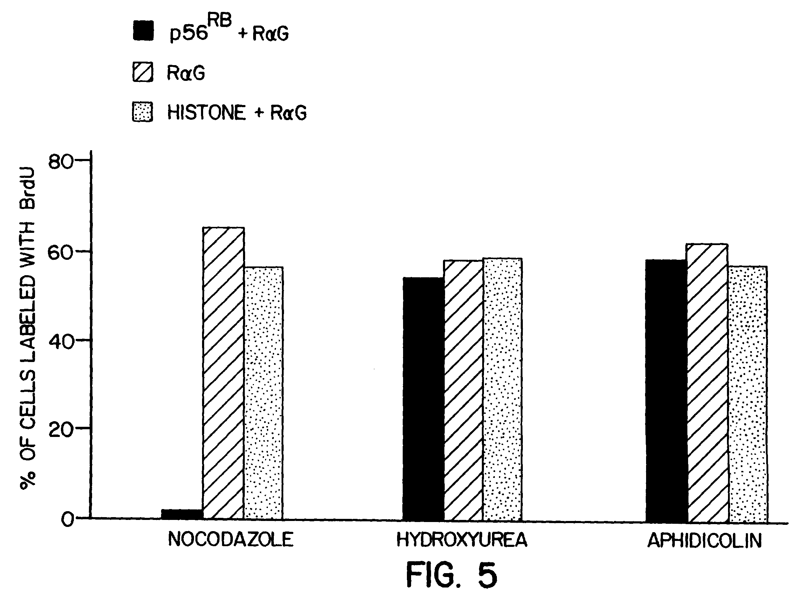

The dose dependence of the p56^{RB} effect was also measured. Diluted aliquots of p56^{RB} were injected into synchronously growing cells in early G1 phase. FIG. 4 graphically shows the dependence of cell cycle arrest on the dose of p56^{RB}. Saos-2 cells were co-injected 6-8 hours after release from nocodazole treatment with the indicated concentrations of p56^{RB} and 1 mg/ml of RαG. After 24 hours of incubation in growth medium with BrdU, cells were fixed and stained for BrdU and RαG. The histogram indicates the percentage of injected cells which incorporated BrdU. Each value represents at least 150 injected cells. As shown in FIG. 4, 5-fold dilution of p56^{RB} from the original concentration diminished the inhibitory activity while aliquots diluted by up to 2-fold still retained the inhibitory effect. Therefore, the block of entry into S phase by p56^{RB} was dependent on the amount of protein injected. The threshold for block of BrdU incorporation was between 0.2 and 0.5 mg/ml of injected p56^{RB}.

To demonstrate that the inhibition of entry into S phase was caused by RB protein, we attempted to alleviate the block with reagents that bound specifically to p56^{RB}. The p56^{RB} was mixed with 1 mg/ml solutions of rabbit polyclonal antibodies .495, .47, or R2 (FIG. 1). These antibodies were raised against unique RB fusion proteins, and recognized p56^{RB} on Western blots (31, 49, 56). Injection of the mixture of p56^{RB} and .495 resulted in BrdU incorporation in about 30% of injected cells, compared to an almost total lack of incorporation in cells injected with p56^{RB} alone (Table 2). Cells co-injected with p56^{RB} and antibody .47 incorporated BrdU about 16% of the time. Co-injection of p56^{RB} and antibody R2 also exhibited a lessened inhibitory activity although the effect was not as dramatic as with antibodies .47 or .495. Because antibodies that bind RB protein diminished the inhibitory effect of p56^{RB}, the block to BrdU incorporation was due specifically to RB protein.

### EXAMPLE III

### BrdU Incorporation is not Inhibited by p56^{RB} if Injected in S-phase

The lack of BrdU incorporation observed after injection of RB protein in early G1 phase could be explained by inhibition of DNA synthesis. To test this, Saos-2 cells were arrested in early S phase by treatment with aphidicolin and then injected with p56^{RB}. As shown graphically in FIG. 5, BrdU incorporation is not inhibited by p56^{RB} if it is injected in the S phase. Saos-2 cells were arrested at G1/S by treatment with hydroxyurea or aphidicolin and were then injected.

After injection, cells were released from arrest and incubated with BrdU for 6-8 hours (hydroxyurea) or 4-6 hours (aphidicolin) at which time the cells were fixed and stained. Cells were also arrested in mitosis by treatment with nocodazole. After release, cells were collected and injected about 6 hours later. Following injection, cells were incubated with BrdU for 24 hours and then fixed and stained.

The protein preparations used for injection are indicated. The percentage of injected cells that stained for BrdU after the respective labeling period is shown. After injection, cells were released from aphidicolin treatment and incubated with BrdU for 4-6 hours, fixed, and immunostained for BrdU and RαG as described. In contrast to cells injected in early G1, approximately 60% of p56^{RB} injected, aphidicolin arrested cells stained positively for BrdU incorporation. The percentage of cells entering S phase and the intensity of BrdU staining was similar to cells injected with histone and RαG, or RαG alone. To control for any possible lag period of the function of RB protein after injection, aphidicolin arrested cells injected with p56^{RB} were incubated for an additional 6 hours prior to release and labeling with BrdU. Despite a somewhat lower intensity of BrdU staining, which was probably caused by the prolonged incubation with aphidicolin itself, the percentage of p56^{RB} injected cells that incorporated BrdU was comparable to that of cells injected with RαG alone.

Similar experiments were performed by arresting cells with hydroxyurea rather than aphidicolin. Hydroxyurea arrests cells near the G1/S boundary at a point distinct from aphidicolin arrest. Saos-2 cells were arrested near G1/S by treatment with hydroxyurea and then were injected with p56^{RB}. After injection, cells were released form hydroxyurea treatment and labeled for 6 to 8 hours with BrdU. Again, injection of p56^{RB} had no detectable inhibitory effect on BrdU incorporation (FIG. 5). The percentage of cells synthesizing DNA was the same whether injecting with p56^{RB} and RαG, histone and RαG, or RαG alone.

The results were consistent with observations of asynchronously growing cells injected with RB protein, and implied that DNA synthesis, per se, was not blocked significantly by p56^{RB}. Therefore, inhibition of DNA synthesis could not explain the complete lack of BrdU incorporation seen upon injection of p56^{RB} into synchronized cells in early G1 phase.

### EXAMPLE IV

### RB Protein Blocks Progression Through the G1 Phase at a Specific Point

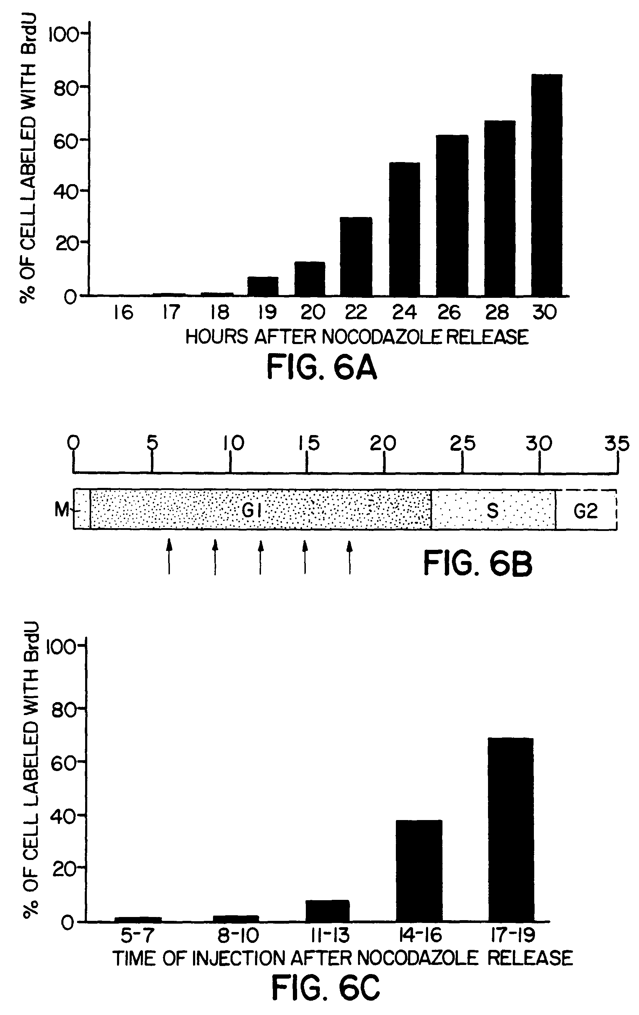

Given the results of the above experiments, the inhibitory effect of p56^{RB} must have been due to a block in progression through the G1 phase. To define the position of this arrest within the G1 phase, a careful calibration of the time of onset of S phase in the cell cycle of Saos-2 cells was first determined. FIGS. 6A, 6B and 6DC graphically depict the dependence of cell cycle arrest on the time period of injection for p56^{RB}. With regard to FIG. 6A, Saos-2 cells were arrested in mitosis by treatment with nocodazole. After release from arrest, the cells were incubated for the indicated number of hours in the presence of BrdU, then fixed and stained with an anti-BrdU antibody. The histogram indicates the percentage of counted cells that stained for BrdU. Each value represents at least 200 cells.

As shown in FIG. 6A, uninjected cells began to stain for BrdU at approximately 20 hours after the release. By 30 hours virtually 80 to 90 percent of cells had incorporated BrdU. Based on this, a time-course experiment in which nocodazole treated cells were injected with p56^{RB} at various times after release from arrest was then performed. FIG. 6B depicts the time of onset of S phase in Saos-2 cells, in hours, after release from nocodazole arrest in mitosis. The length of G1 phase is about 22 to 24 hours. The length of S phase is about 7 to 8 hours. The length of the G2 phase has not been precisely determined. The arrows indicate the time points of injection that are used for the experiment described in 6C.

With regard to FIG. 6C, Saos-2 cells were co-injected with 1 mg/ml p56^{RB} and RαG at the indicated times, in hours, after release from nocodazole treatment. Incubation was continued after injection in growth medium with BrdU until 30 hours after the original release from nocodazole. After BrdU labeling, cells were fixed and stained for BrdU and RαG. The histogram shows the percentage of p56^{RB} injected cells which incorporated BrdU. Each value represents at least 200 injected cells.

Very few cells entered S phase at 30 hours after release if injected with p56^{RB} 5-10 hours after release from nocodazole (FIG. 6C). A significant number of cells incorporated BrdU if injected with p56^{RB} after 14-16 hours post-arrest. If injected 17-19 hours after release, p56^{RB} had no detectable inhibitory effect on entry into S phase.

Given that S phase began between 22 and 24 hours after release from nocodazole, the results suggested that cells become refractory to the inhibitory effect of injected RB protein about 6-10 hours prior to the G1/S transition. This confined the notion that the inhibitory effect of RB protein on the cell cycle was due to a block in progression through the G1 phase rather than a block of DNA synthesis itself. Furthermore, the observations implied that RB may function at a specific, relatively early time point within the G1 phase.

### EXAMPLE V

### SV40 T Antigen Relieves the Blocked Cell Cycle Progression by p56 RB

As a test of the hypothesis that SV40 T-antigen can functionally inactivate RB protein upon binding, we compared the effect of p56^{RB} injection on G1 progression in the presence or absence of T-antigen. A 1mM T peptide solution which was capable of binding to p56^{RB}, was mixed with an equal volume of p56^{RB} and injected into synchronized cells in early G1 phase. At this concentration, the peptide was at a 100 to 200-fold molar excess over the RB protein. About 28% of synchronized cells injected with the p56^{RB}/T peptide mixture entered S during the labeling period (Table 3). If the T peptide concentration was increased to 5 mM, or a 500 to 1000-fold molar excess, approximately 70% of injected cells could incorporate BrdU. By contrast, almost none of the cells progressed into S phase after injection with p56^{RB} and a 500 to 1000-fold molar excess of a carboxy-terminal p53 peptide that did not bind p56^{RB}.

The effects of injection of p56^{RB} on African Green monkey kidney cell lines CV-1 and COS-7 were also compared. COS-7 cells were chosen since they were derived from CV-1 cells by transformation with an origin-defective SV-40 mutant, and they expressed a high level of T-antigen. Therefore, they can serve as a good system for testing the effect, if any, of the presence of endogenous T-antigen on the activity of the injected RB protein. The percentage of synchronized CV-1 cells injected with p56^{RB} in early G1 phase that incorporated BrdU was at least 5-fold lower than cells injected with RαG alone (Table 3). Synchronized COS-7 cells, however, were not inhibited at all from progressing into S phase. These results indicated that the presence of endogenous T-antigen, or co-injection with a T-antigen peptide, could neutralize the inhibitory activity of RB protein. Thus, those skilled in the art can utilize this system to screen RB protein fragments to identify those fragments capable of blocking cell cycle progression by comparing the percentage of CV-1 to COS-7 cells entering S phase in the presence of the RB protein fragment.

The foregoing results provide functional evidence which indicates that RB protein can act by inhibiting progression through the cell cycle. Injection of p56^{RB} does not detectably inhibit DNA synthesis per se, since aphidicolin or hydroxyurea arrested cells are not affected, but rather blocks progression through the G1 phase. This inhibition of cycle progression is dose-dependent, is not due to general toxicity to the cells, and is specific for RB protein because antibodies that bind specifically to p56^{RB} can attenuate its activity. The cycling cell is sensitive to RB protein inhibition until roughly 6-10 hours before the onset of DNA replication; after this time, exogenously added p56^{RB} no longer inhibits progression into S phase. Assuming there is a small lag period between p56^{RB} injection and its appearance in an active form in the nucleus, it may be concluded that RB protein inhibits progression to S phase at a critical point in G1 phase. The 6-10 hour time window prior to DNA synthesis may be analogous to the time period following the G1 restriction point, a point of irreversible commitment to S phase in mammalian cell lines 47.

The time window defined may not correspond precisely to the time point when RB protein functions under normal physiological conditions. The time it takes for injected p110^{RB} or p56^{RB} to appear in an active configuration and/or location is not clear. However, RB proteins are transported to the nucleus within 15 minutes after cytoplasmic injection, and continued incubation of p56^{RB} injected cells with aphidicolin for several hours prior to release and labeling does not decrease the percentage of cells which incorporate BrdU. These observations suggest that the time it takes RB protein to become functional after injection may be short.

The transition from an active form of p110^{RB} to an inactive form has been assumed to occur at the G1/S boundary since the protein undergoes extensive phosphorylation at this point. However, because of the foregoing, an earlier time period, which seems to be critical for p56^{RB} activity has been defined. Thus, RB may be involved in a regulatory decision which the cell makes at a point about 6-10 hours prior to the G1/S transition. The RB protein can be phosphorylated at multiple sites both in vivo and in vitro, yet it is not yet clear which phosphorylation sites are important for RB function. Subtle increases in phosphorylation of p110^{RB} during G1 phase may be responsible for the cells' transition from an RB-responsive to a non-responsive state. Some phosphorylation of p110^{RB} has been observed in the G1 phase, although this may have been due to incompletely synchronized cells.

The carboxy-terminal 56 kiloDaltons of RB is sufficient to inhibit progression through the G1 phase. This indicates that the carboxy terminal half of p110^{RB} is, in fact, a functional domain with respect to its effect on the cell cycle. Two biochemical activities have been ascribed to the carboxy-terminal half of p110^{RB}. DNA binding and protein binding. Based on findings to date, the sequence specificity of RB binding to DNA is low; although RB binds with slightly higher affinity to DNA with high G/C content, no particular sequence is strongly preferred. On the other hand, specificity has been observed in binding of RB to proteins. The transforming proteins of several DNA transforming viruses as well as a subset of cellular proteins bind to the same domain of p110^{RB} and therefore can compete with one another for RB protein binding. This region is where the majority of naturally occurring inactivating mutations of RB are located. It seems likely, then, that the block to progression of G1 phase by RB protein is dependent on specific protein-protein interactions.

Mixing p56^{RB} with a T-antigen derived peptide, which can bind p56^{RB}, eliminates its inhibitory consequences. Co-injection of p56^{RB} with a T peptide containing a single amino acid substitution at a 500 to 1000-fold molar excess can also attenuate, to some extent, the block to cell cycle progression. Although the amino acid substitution lowers its affinity for RB protein in vitro, this mutant T peptide may still bind p56^{RB} in vivo when present in vast molar excess. In contrast, the injection of a totally irrelevant p53 peptide had no effect on the inhibitory activity of RB protein, which thus substantiated the specificity of the interaction observed between RB protein and T-antigen peptide. CV-1 cells injected in early G1 with p56^{RB} are arrested before S phase while COS-7 cells, which express high levels of T-antigen, are not arrested, however. These observations, taken together, indicate that T-antigen binding does have functional consequences for RB protein. Since the injected p56^{RB} is unphosphorylated, the test results are consistent with the hypothesis that the active form of RB protein, with respect to its inhibition of cell proliferation, is the unphosphorylated form. Therefore, the transforming proteins of some DNA tumor viruses, including SV40 T-antigen and adenovirus E1A, may promote cell growth, at least in part, by binding and inactivating underphosphorylated p110^{RB}. The immortalization of cells and the induced escape from quiescence upon expression of these transforming proteins are phenotypes consistent with deregulation of the cell cycle.

Given that the carboxy-terminal half of p110^{RB} is biologically active, the question remains as to the function of the amino terminal half of the protein. Sequences within this region may be required for the proper phosphorylation of p110^{RB}. In murine cells, polypeptides similar to p56^{RB} are not hyperphosphorylated. Also, several consensus sites for the cdc2/MPF kinase are present within this region. It follows that the amino terminal half of p110^{RB} may contain a regulatory domain which can modulate RB function. Release of p56^{RB} block to G1 progression does not occur until 3-4 days after injection; this lengthy arrest period may be due to the inability of the cell to regulate RB protein in a normal manner, or to the relatively large amount of RB protein injected. A comparison of the activity in this assay of different RB proteins with mutations in phosphorylation sites would aid in resolving this issue. If p56^{RB}is constitutively active, mutations creating such polypeptides would be expected to inhibit cell proliferation and thus place cells at a selective disadvantage. This may explain the lack of naturally occurring mutants within the amino terminal half of RB.

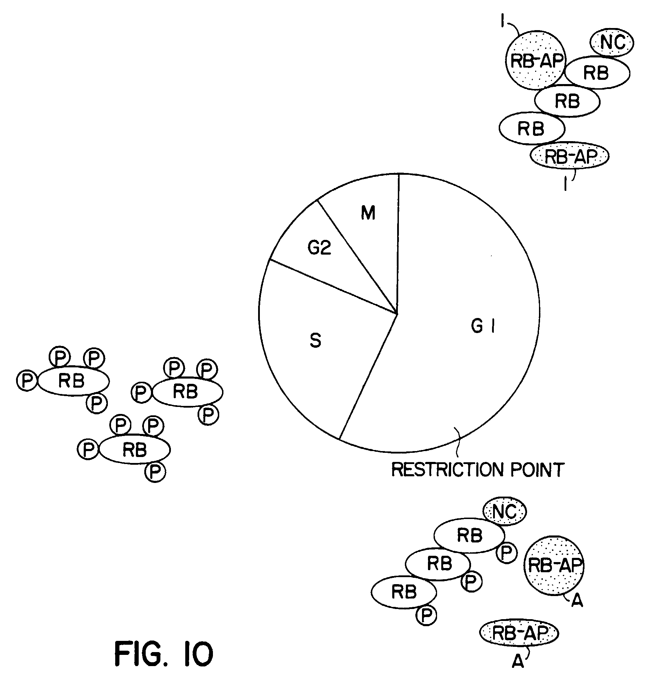

The results described here indicate that RB participates in control of cell cycle progression by acting at a control point within the G1 phase. This point is significantly earlier than the observed hyperphosphorylation of p110^{RB} as cells enter S phase. Thus, it is reasonable to propose a model which incorporates the test results as well as other known properties of p110^{RB} (FIG. 10). Since RB has been shown to associate with several cellular proteins, and the protein binding domains of RB are sufficient to block cell cycle progression, it seem highly possible that the binding of cellular proteins is critical for the observed RB function. The model suggests that RB participates in regulation of the cell cycle by sequestering cellular proteins vital for DNA synthesis and/or cycle progression at a particular time period during the G1 phase. Inactivation of p110^{RB} by mutation, by phosphorylation at key sites, or by binding with transforming proteins is hypothesized to release these cellular proteins, permitting them to provide functions that are necessary for continuation of the cell cycle. Once cells commit themselves to continuation of the cell cycle by release of these proteins, p110^{RB} can no longer effectively inhibit their function and therefore the block to cell cycle progression is relieved.

Sub-nuclear localization of RB may be crucial to its ability to sequester other proteins in an inactive form. RB protein segregates with cellular replication proteins to sites of Herpes virus DNA replication upon infection, a situation where normal regulation of the cell cycle is subverted. In G1, the underphosphorylated form of p110^{RB} is tightly associated with a particular nuclear locale. Purified p110^{RB} also tends to polymerize, perhaps explaining the difficulty in maintaining its solubility at high concentrations, and it has limited homology to a certain class of intermediate filament proteins. These observations suggest that p110^{RB} may contribute to some structural component of the nucleus, and that association of p110^{RB} with this component is necessary for its inhibition of cell cycle progression. The hyperphosphorylation would presumably free p110^{RB} from its sub-nuclear location allowing RB to provide some other function, or to reset its ability to sequester other proteins during the subsequent cell cycle. The model predicts that regulation of RB activity could be accomplished by specifying its nuclear location and/or the cellular proteins to which it binds.

The biological consequence of complete loss of RB function is generation of retinoblastoma and perhaps some other tumors. involvement of RB in the cell cycle provides a means to explain this phenomenon. RB may act to halt progression through G1 phase until the cell receives proper signals for commitment to continuation of the cell cycle. Thus, loss of RB function may contribute to tumorigenesis in different tissues by permitting unscheduled cell proliferation.

In view of the foregoing, it can be seen that the RB gene product is a nuclear phosphoprotein which undergoes changes in phosphorylation status in synchrony with the cell cycle. To test whether RB regulates cell cycle progression, purified RB proteins, either full-length or a truncated form containing the T-antigen binding region, were injected into cells and the effect on entry into S phase determined. Synchronized cells injected early in G1 with either protein are inhibited from progressing into S phase. This effect is antagonized by co-injection with antibodies directed against RB. Injection of RB protein into cells arrested at the B1/S boundary or 6-10 hours before the end of G1 has no effect on BrdU incorporation suggesting that RB protein does not apparently perturb DNA synthesis in S phase. These results provide direct evidence that RB may regulate cell proliferation by restricting cell cycle progression at a specific point in G1, and they also establish a biological assay for the activity of RB protein, Co-injection of RB protein with a T-antigen peptide, or injection into cells expressing T-antigen does not prevent cells from progressing into S phase. This experiment substantiates the hypothesis that T-antigen binding has functional consequences for RB protein.

**Table 1**

| Microinjection of Asynchronously Growing Cells | | | |
|---|---|---|---|
| Protein Sample | Total Number of Injected Cells^{a} | Total Number of BrdU Stained Cells^{b} | % Injected Cells Entering S Phase |
| pp110^{RB} | 1094 | 416 | 38 |
| P56^{RB} | 1315 | 408 | 31 |
| RαG | 1725 | 707 | 41 |
| Histone | 106 | 42 | 40 |
| Uninjected | | | 41 |

| | | | |
|---|---|---|---|
| ^{a} The total number of injected cells (Texas Red positive) counted from at least five independent experiments, except for the Histone injection which represents one experiment. | | | |
| ^{b} The number of injected cells which incorporated BrdU as evidenced by uniform staining with a fluorescein linked anti-BrdU antibody. Cells were incubated in growth media with BrdU for 4 hours prior to staining. | | | |

**Table 2**

| Microinjection of synchronously Growing Cells in Early G1 | | | | |
|---|---|---|---|---|
| Cell Line | Protein | Number of Injected Cells^{a} | Number of BrdU Stained Cells^{b} | % of Injected Cells Entering S phase |
| | | | | |

| Saos-2 | | | | |
|---|---|---|---|---|
| | p110^{RB} ^{c} | 256 | 8 | 3 |
| | RαG^{c} | 270 | 173 | 64 |
| | p56^{RB} | 388 | 12 | 3 |
| | RαG | 295 | 202 | 68 |
| | Histone | 595 | 333 | 56 |
| | Uninjected | | | 84 |
| | p56^{RB}^{d} | 247 | 10 | 4 |
| | p56^{RB}^{d}+.495 | 80 | 24 | 30 |
| | p56^{RB}^{d}+.47 | 596 | 95 | 16 |
| | p56^{RB}d+R2 | 712 | 43 | 6 |

| SR-40 | | | | |
|---|---|---|---|---|
| | p56^{RB} | 89 | 3 | 3 |
| | Histone | 201 | 105 | 52 |
| | RαG | 134 | 74 | 55 |

| | | | | |
|---|---|---|---|---|
| ^{a} The total number of injected cells (Texas Red positive) counted from at least three independent experiments. | | | | |
| ^{b} The number of injected cells which incorporated BrdU as evidenced by uniform staining with a fluorescein linked anti-BrdU antibody. Cells were incubated in growth media with BrdU for 24 hours after injection and prior to staining. | | | | |
| ^{c} Injections were performed in a buffer containing 10% glycerol. | | | | |
| ^{d} p56RB was injected at a concentration of about .3-.5 mg/ml, rather than 1 mg/ml, in these experiments. | | | | |

**Table 3**

| SV40 T-antigen relieves the block to G1 progression by p56^{RB} | | | | |
|---|---|---|---|---|
| Cell Line | Protein^{a} | Number of Injected Cells^{b} | Number of BrdU Stained Cells^{c} | % of Injected Cells Entering S phase |
| | | | | |

| Saos-2 | | | | |
|---|---|---|---|---|
| | p56^{RB} | 247 | 10 | 4 |
| | p56^{RB}+T | 724 | 203 | 28 |
| | p56^{RB}+p53 | 727 | 7 | 1 |

| CV-1 | | | | |
|---|---|---|---|---|
| | p56^{RB} | 459 | 46 | 10 |
| | Histone | 471 | 259 | 55 |
| | RαG | 332 | 176 | 53 |

| COS-7 | | | | |
|---|---|---|---|---|
| | p56^{RB} | 257 | 224 | 87 |
| | Histone | 191 | 159 | 83 |

| | | | | |
|---|---|---|---|---|
| ^{a} All proteins were injected at concentrations of about 1 mg/ml, except for p56^{RB} in Saos-2 cells which was injected at about .3-.5 mg/ml. | | | | |
| ^{b} Cell lines are microinjected 6-8 hours after release from nocodazole treatment. The total number of injected cells (Texas Red positive) is indicated. | | | | |
| ^{c} Cells were incubated in growth media with BrdU for 24 hours after injection and prior to staining. | | | | |

While particular embodiments of the present invention have been disclosed, it is to be understood that various different modifications are possible and are contemplated within the scope of the appended claims. There is no intention, therefore, of limitations to the exact abstract or disclosure herein presented.

## Claims

1. Use of a C-terminal biologically active fragment of p110^{RB} of 56 kD for the preparation of a medicament for controlling cell cycle progression in a cell lacking a functional retinoblastoma tumor suppressor gene.

2. The use according to claim 1, wherein the C-terminal fragment comprises amino acid 379 to amino acid 928 as shown in Figure 8.

3. The use of claims 1 and 2, wherein the medicament is for the treatment of a pathology characterized by cell cycle progression through the absence of a functional retinoblastoma tumor suppressor protein.

4. A method of controlling cell cycle progression in a cell in vitro, the cell lacking a functional retinoblastoma tumor suppressor gene, comprising in vitro introducing into the cell to be controlled a C-terminal biologically active fragment of p110^{RB} of 56 kD during the G phase of the cell cycle.

5. The method of claim 4, wherein the C-terminal fragment comprises amino acid 379 to amino acid 928 as shown in Figure 8.

## Patentansprüche

1. Verwendung eines C-terminalen biologisch aktiven Fragments von 56 kD des p110^{RB} für die Herstellung eines Arzneimittels zur Kontrolle der Zellzyklus-Progression in einer Zelle, der ein funktionelles Retinoblastom-Tumor-supprimierendes Gen fehlt.

2. Verwendung nach Anspruch 1, bei der das C-terminale Fragment die in Abbildung 8 gezeigten Aminosäuren 379 bis 928 umfaßt.

3. Verwendung nach den Ansprüchen 1 und 2, bei der das Arzneimittel zur Behandlung von Pathologien bestimmt ist, die durch Zellzyklus-Progression infolge des Fehlens eines funktionellen Retinoblastom-Tumor-supprimierenden Gens gekennzeichnet sind.

4. Verfahren zur Kontrolle der Zellzyklus-Progression in einer Zelle *in vitro*, wobei der Zelle ein funktionelles Retinoblastom-Tumor-supprimierendes Gen fehlt, wobei das Verfahren die *in vitro*-Einführung eines C-terminalen biologisch aktiven Fragments von 56 kD des p110^{RB} in die zu kontrollierende Zelle während der G-Phase des Zellzyklus umfaßt.

5. Verfahren nach Anspruch 4, bei dem das C-terminale Fragment die in Abbildung 8 gezeigten Aminosäuren 379 bis 928 umfaßt.

## Revendications

1. Utilisation d'un fragment C-terminal biologiquement actif de p110^{RB} de 56 kD pour la préparation d'un médicament destiné à contrôler la progression du cycle cellulaire dans une cellule dépourvue d'un gène supresseur de tumeur rétinoblastome fonctionnel.

2. Utilisation selon la revendication 1, dans laquelle le fragment C-terminal comprend les acides aminés 379 à 928 comme présenté la figure 8.

3. Utilisation des revendications 1 et 2, dans laquelle le médicament est destiné au traitement d'une pathologie caractérisée par une progression du cycle cellulaire en l'absence d'une protéine suppresseur de tumeur rétinoblastome fonctionnelle.

4. Méthode de contrôle de la progression du cycle cellulaire dans une cellule in vitro, la cellule étant dépourvue d'un gène suppresseur de tumeur rétinoblastome fonctionnel, comprenant l'introduction in vitro dans la cellule à contrôler d'un fragment C-terminal biologiquement actif de p110^{RB} de 56 kD pendant la phase G du cycle cellulaire.

5. Méthode de la revendication 4, dans laquelle le fragment C-terminal comprend les acides aminés 379 à 928 comme présenté à la figure 8.
